# EUROPÄISCHE PATENTSCHRIFT

(11) **EP 3 254 728 B1**
(45) Veröffentlichungstag und Bekanntmachung des Hinweises auf die Patenterteilung: **30.09.2020**
(21) Anmeldenummer: 17174198.6
(22) Anmeldetag: 02.06.2017
(51) Int. Cl.: A61N 1/375, B81B 7/00, H01L 23/10, H05K 5/06

(54) **STOFFSCHLÜSSIGE METALLISCHE VERBINDUNG BASIEREND AUF EINER GALVANISCHEN ABSCHEIDUNG**
INTEGRAL METALLIC JOINT BASED ON ELECTRODEPOSITION
LIAISON MÉTALLIQUE PAR LIAISON DE MATIÈRE SUR BASE D'UNE SÉPARATION GALVANIQUE

(30) Priorität: 08.06.2016 DE 102016110539
(43) Veröffentlichungstag der Anmeldung: 13.12.2017
(73) Patentinhaber: BIOTRONIK SE & Co. KG, 12359 Berlin (DE)
(72) Erfinder: Hauer, Marc, 8610 Uster (CH)
(74) Vertreter: Galander, Marcus

(56) Entgegenhaltungen:
- WO-A1-01/43517
- DE-A1-102005 053 765
- US-A1- 2002 019 669
- US-A1- 2009 289 349

## Beschreibung

Die Erfindung betrifft eine elektronische Baugruppe, die eine aus mindestens zwei Hüllenteilen zusammengefügte Umhüllung aufweist, und beispielsweise auch ein implantierbares medizinelektronisches Gerät. Sie betrifft des Weiteren ein Verfahren zur Herstellung einer solchen elektronischen Baugruppe.

Von elektronischen Geräten oder Baugruppen wird in gewissen Anwendungssituationen völlige ("hermetische") Dichtigkeit verlangt. Hierzu zählen Geräte, die unter aggressiven Umweltbedingungen oder auch in einem lebenden Körper dauerhaft zuverlässig arbeiten müssen, also u. a. implantierbare medizinelektronische Geräte. Gelegentlich wird aber auch von speziellen Baugruppen in größeren Geräten oder Vorrichtungen eine solche hermetische Dichtigkeit verlangt.

Es gibt erprobte Verfahren, um derart dichte elektronische Baugruppen bzw. Geräte mit vertretbarem Aufwand herzustellen. Hierzu zählen - je nach Material und geometrischer Konfiguration der Geräteumhüllung - Schweißverfahren (insbesondere Laserschweißverfahren), Lötverfahren (insbesondere Hartlötverfahren), Sinterverfahren und ähnliche. Bei all diesen Verfahren wird die Baugruppe bzw. das Gerät jedenfalls abschnittsweise einer relativ hohen thermischen Belastung ausgesetzt, die entsprechende thermische Spannungen und ggf. auch gewisse Schädigungen der verwendeten Materialien zur Folge hat. Diese thermischen Spannungen oder gewissen Schädigungen sind grundsätzlich unerwünscht, werden jedoch in der Regel hingenommen. Als grundsätzlicher Nachteil dieser Verfahren ist anzusehen, dass die unvermeidliche thermische Belastung das Spektrum der einsetzbaren Materialien von vornherein einschränkt.

Die Dokumente US 2009 289349 A1, DE 10 2005 053 765 A1 und WO 01/43517 A1 zeigen jeweils eine elektronische Baugruppe mit einer aus zwei Hüllenteilen zusammengefügten Umhüllung, die mittels einer galvanisch abgeschiedenen Metallschicht hermetisch dicht verschlossen ist.

Es ist daher Aufgabe der Erfindung, eine unter diesen Aspekten verbesserte elektronische Baugruppe und ein Verfahren zur Herstellung einer solchen bereitzustellen, wobei insbesondere thermische Belastungen bei der Herstellung der Hülle und damit einhergehende Beschränkungen der Materialwahl weitestgehend vermieden werden sollen.

Diese Aufgabe wird in ihrem Vorrichtungsaspekt durch eine elektronische Baugruppe mit den Merkmalen des Anspruchs 1 und in ihrem Verfahrensaspekt durch ein Verfahren mit den Merkmalen des Anspruchs 12 gelöst. Zweckmäßige Fortbildungen des Erfindungsgedankens sind Gegenstand der jeweiligen abhängigen Ansprüche.

Die Erfindung schließt den Gedanken ein, Spalten zwischen Hüllenteilen der Baugruppe bzw. des Gerätes in einem "kalten" Verfahren und gleichwohl stoffschlüssig zu versiegeln. Sie schließt, angesichts der bekannten Probleme der dauerhaften Aufrechterhaltung hermetischer Dichtigkeit an den Grenzflächen miteinander verbundener Teile, des Weiteren den Gedanken ein, eine Überbrückung bzw. Überdeckung des Spaltbereiches, unter Einschluss der angrenzenden Hüllenteil-Oberflächen, vorzusehen. Schließlich gehört zur Erfindung der Gedanke, diese Überbrückung bzw. Überdeckung mit einer galvanisch mindestens auf die an den Spalt angrenzenden Abschnitte der benachbarten Hüllenteile abgeschiedenen Metallschicht vorzunehmen.

Ein wesentlicher Vorteil der vorgeschlagenen Lösung besteht im völligen Fortfall jeglicher thermischer Belastung und somit auch der hiermit verbundenen Nachteile, insbesondere thermischer Spannungen und einer Einschränkung der Materialauswahl. Hierbei ist speziell darauf hinzuweisen, dass sich gemäß dem vorgeschlagenen Konzept für den Aufbau der Baugruppen-Hülle auch Materialien mit stark unterschiedlichem thermischen Ausdehnungskoeffizienten (CTE) und Materialien mit sehr geringer thermischer Belastbarkeit einsetzen lassen. Je nach Anwendungsfall, können weitere Vorteile hinzutreten, etwa der Wegfall einer thermischen Belastung der in der Baugruppenhülle aufgenommenen Bauelemente bzw. -teile, eine Reduzierung des Energieaufwandes bei der Herstellung und somit auch der Herstellungskosten usw.

In einer praktisch wichtigen Ausführung ist die Baugruppe ausgebildet als implantierbares medizinelektronisches Gerät, insbesondere Herzschrittmacher, Kardioverter, Cochlearimplantat oder Medikamentendosierpumpe. Für derartige Geräte ist hermetische Dichtigkeit ein grundlegendes Erfordernis, und die herkömmlichen thermischen Verfahren beispielsweise zur Bildung der sogenannten Durchführung zwischen dem Gehäuse-Hauptteil und dem Kopfteil (Header) eines Schrittmachers oder Kardioverters, teilweise aber auch zur Bildung des Gehäuse-Hauptteils, hatten bisher signifikante Einschränkungen bei der Materialwahl zur Folge. Realisierungen der vorgeschlagenen Baugruppe als vollständig hermetisch dichtes Gerät sind aber nicht auf implantierbare oder allgemeiner medizinelektronische Geräte beschränkt, sondern derartige Geräte können auch bei der Prozesssteuerung in Explorations- oder Chemieanlagen, in der Flugzeugtechnik und Raumfahrt etc. von Bedeutung sein.

In einer weiteren Ausführung ist die elektronische Baugruppe ausgebildet als hermetisch dichte und optional auch EMI-dichte Komponente eines nicht insgesamt dichten elektronischen Gerätes. Insbesondere kann es sich hierbei um mit einem Deckel bzw. einer Haube versehene Bereiche einer bestückten Leiterplatte oder eines anderen Schaltungssubstrates handeln, an die hinsichtlich der Dichtigkeit höhere Anforderungen als an andere Bereiche bzw. Baugruppen des entsprechenden Gerätes gestellt werden.

In einer weiteren Ausführung ist mindestens eines der benachbarten Hüllenteile ein Metallteil mit unter der abgeschiedenen Metallschicht metallisch blanker Oberfläche. Das entsprechende Metallteil kann also eines sein, das als solches nicht zur Ausbildung einer natürlichen Oxidschicht (die die galvanische Beschichtung verhindern würde) neigt, also insbesondere Gold oder ein anderes Edelmetall. Es kann aber auch unmittelbar vor der galvanischen Abscheidung einem Reinigungsvorgang unterzogen und/oder vorab mit einem nichtkorrodierenden Metall verbunden oder überzogen worden sein.

In einer weiteren Ausführung ist mindestens eines der benachbarten Hüllenteile ein Kunststoffteil, insbesondere eine Leiterplatte, oder ein Keramikteil, insbesondere ein Dickschicht-Substrat, mit einer zusätzlichen Metallisierungsschicht unter der abgeschiedenen Metallschicht. Die zusätzliche Metallisierungsschicht (die auch als aufgesetztes oder angefügtes zusätzliches Metallteil realisiert sein kann) sorgt für die erforderliche elektrische Leitfähigkeit für die nachfolgende Metallabscheidung aus dem Elektrolyten. Sie ist grundsätzlich nur in dem Bereich oder entlang einer Kante erforderlich, wo die abdichtende Metallschicht tatsächlich gebildet werden soll.

Erfindungsgemäß sind die einander benachbarten Hüllenteile mit einem Klebstoff verbunden, der den Spaltbereich weitgehend ausfüllt und zumindest an dessen Außenseite leitfähig ist. Ein homogen leitender Klebstoff wird eingesetzt, da dies technologisch einfacher ist. Anwendungsbezogen kann beim Einsatz einer Klebeverbindung davon abgesehen werden, ein elektrisch isolierendes Hüllenteil vorab als solches leitfähig zu machen. Dies wäre insbesondere dann möglich, wenn leitfähiger Klebstoff auch auf den an den Spalt angrenzenden Oberflächenabschnitt des isolierenden Hüllenteils aufgetragen wird, diesen also gewissermaßen "metallisiert".

In weiteren Ausführungen der Erfindung ist vorgesehen, dass die galvanisch abgeschiedene Metallschicht eine dichte, porenfreie Schicht bildet und so die Diffusion unterbindet. Je nach Anforderungsprofil kann es sich dabei um inerte Metalle wie, z. B. Gold, Platin oder Palladium oder nicht inerte Metalle wie z. B. Cu oder Ni handeln. In einer speziellen Ausführungsform kann die Abscheidung auch mehrere Metalle als Legierung oder Schichtfolge beinhalten. Typische Metallschichtdicken bewegen sich zwischen 0.1 - 100 µm.

Bei der weiter oben erwähnten Ausführung als medizinelektronisches Gerät kann insbesondere die galvanisch abgeschiedene Metallschicht auf einer Durchführung des medizinelektronischen Gerätes vorgesehen sein und insbesondere den Spaltbereich zwischen einem Flanschteil und einem Leiterelement der Durchführung überbrücken und abdichten. Speziell kann hierbei vorgesehen sein, dass das Flanschteil aus Titan besteht und partiell mit einem Edelmetall-Zusatzteil oder einer Edelmetallschicht versehen ist und das Leiterelement ein Kunststoff- oder Keramiksubstrat aufweist und partiell mit einer Edelmetallschicht versehen ist.

Des Weiteren kann bei einem solchen implantierbaren Gerät auch das eigentliche Gehäuse erfindungsgemäß aufgebaut sein, also insbesondere aus zwei Gehäuseschalen, der Spaltbereich mit einer galvanisch abgeschiedenen Metallschicht überdeckt und somit hermetisch dicht verschlossen ist.

In der ebenfalls weiter oben erwähnten Realisierung als einzelne Baugruppe eines größeren Gerätes kann eines der benachbarten Teile eine Leiterplatte oder ein Dickschichtsubstrat und das andere Teil ein metallisches Deckelteil sein und die Leiterplatte oder das Dickschichtsubstrat eine entsprechend der Öffhungskontur des Deckelteils konfigurierte partielle Metallbeschichtung als Unterlage für die galvanisch abgeschiedene Metallschicht aufweisen.

Verfahrensaspekte der Erfindung in bevorzugten Ausführungen ergeben sich teilweise aus den weiter oben erläuterten Vorrichtungsaspekten und müssen insoweit hier nicht wiederholt werden. Grundsätzlich lassen sich die wesentlichen Schritte des vorgeschlagenen Verfahrens wie folgt darstellen:
- Bereitstellen zweier für eine galvanische Abscheidung inhärent geeigneter oder vorbereiteter Hüllenteile,
- Anordnen der Hüllenteile in einer vorbestimmten Lagebeziehung zueinander, die einen Spaltbereich dazwischen einschließt, und Fixieren der Lagebeziehung der Hüllenteile, wobei das Fixieren der Lagebeziehung der Hüllenteile deren Verkleben mittels eines homogen leitenden Leitklebers und mindestens teilweise Aushärten des Klebers einschließt,
- Verbringen der in ihrer Lagebeziehung fixierten Hüllenteile in ein Elektrolysebad, und
- Ausführen einer galvanischen Abscheidung im Elektrolysebad mit Verfahrensbedingungen, die die Abscheidung einer den Spaltbereich überbrückenden und hermetisch abdichtenden Metallschicht gewährleisten.

Hierbei ist zu erwähnen, dass die Positionierung der Hüllenteile für die galvanische Beschichtung sinnvollerweise so nahe wie möglich zueinander, also mit möglichst kleinem Spaltbereich, erfolgen wird. Es versteht sich jedoch, dass bei einer Serienproduktion Maßtoleranzen der Hüllenteile zu beachten sind und eine Nachjustierung der Position für jede einzelne Baugruppe bzw. jedes einzelne Gerät mit Blick auf die Kosten normalerweise nicht zweckmäßig sein wird. Hinsichtlich der einzustellenden Verfahrensbedingungen ist zu erwähnen, dass die abgeschiedene Metallschicht hinreichend dick sein muss, um eine dauerhafte hermetische Dichtigkeit im Spaltbereich zu gewährleisten, je nach Anwendungsfall auch bei gewissen mechanischen Beanspruchungen der Baugruppen- bzw. Gerätehülle und/oder bei Temperaturwechselbelastungen im Betrieb.

In Ausführungen der Erfindung wird die galvanische Abscheidung in einem Elektrolysebad ausgeführt, welches einen dichten Schichtaufbau und eine gute Haftung aufweist. Vorzugweise können Elektrolyte zum Einsatz kommen, die eine beschleunigte Abscheidung in einem Spalt im Vergleich zur Oberfläche ermöglichen (z. B. Dow Chemical EVF).

In einer weiteren Ausführung weist die galvanisch abgeschiedene Metallschicht mehrere Metalle in Form einer Legierung und/oder einer Schichtfolge mit jeweils unterschiedlichen Metallen in jeder Teilschicht auf.

In einer noch weiteren Ausführung hat die galvanisch abgeschiedene Metallschicht eine Dicke im Bereich zwischen 0,1 und 100 µm.

Vorteile und Zweckmäßigkeiten der Erfindung ergeben sich im Übrigen aus der nachfolgenden Beschreibung von Ausführungsbeispielen anhand der Figuren. Von diesen zeigen:
- Fig. 1A und 1B: Prinzipskizzen zur Erläuterung eines Beispieles, das keine Ausführungsform der Erfindung darstellt.
- Fig. 2A und 2B: Prinzipskizzen zur Erläuterung eines weiteren Beispiel, das kein Teil der Erfindung ist.
- Fig. 3A und 3B: Prinzipskizzen zur Erläuterung einer grundsätzlichen Ausführung der Erfindung,
- Fig. 4A und 4B: Prinzipskizzen zur Erläuterung eines Beispiels, bei einem implantierbaren medizinelektronischen Gerät, das kein Teil der Erfindung ist,
- Fig. 5A - 5C: Prinzipskizzen zur Erläuterung einer weiteren Ausführung der Erfindung, bei einem implantierbaren medizinelektronischen Gerät,
- Fig. 6A und 6B: Prinzipskizzen zur Erläuterung einer Ausführung der Erfindung, bei einer abgegrenzten elektronischen Baugruppe eines Gerätes, und
- Fig. 7A und 7B: Prinzipskizzen zur Erläuterung eines weiteren Beispieles, bei einer abgegrenzten elektronischen Baugruppe eines Gerätes, das keine Ausführungsform der Erfindung darstellt.

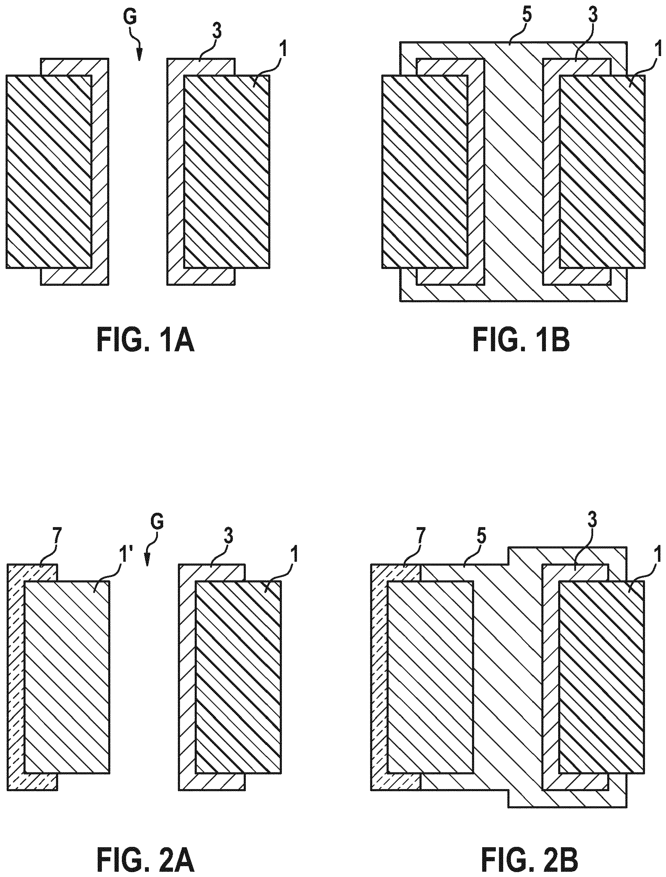

Fig. 1A zeigt schematisch in Art eines Längsschnitts zwei Werkstücke (Hüllenteile einer Gerätehülle) 1, die an ihren einander zugewandten Oberflächen hermetisch dicht miteinander verbunden werden sollen, und Fig. 1B zeigt die Werkstücke (Hüllenteile) im dicht verbundenen Endzustand. Um das Verfahren anwenden zu können, wurden die Hüllenteile 1 vorab jeweils partiell, und zwar vollflächig an den einander zugewandten Oberflächen und zusätzlich in einem Teil ihrer Ober- und Unterseite, mit einer leitfähigen Beschichtung 3 versehen. Sie werden dann mit einem vorbestimmten Spalt G dazwischen relativ zueinander fixiert und in ein Elektrolysebad gebracht, wo galvanisch eine Metallschicht 5 auf allen metallischen Oberflächen abgeschieden wird (aufwächst) und hierbei den Spalt G vollständig und hermetisch dicht verschließt.

Fig. 2A zeigt wiederum zwei vorbehandelte Hüllenteile 1, 1', jedoch mit einer anderen Materialkonfiguration: Das Hüllenteil 1 ist wieder aus einem isolierenden Material (etwa Kunststoff oder Keramik) gefertigt, während das Hüllenteil 1' ein Metallteil ist. Wie bei dem vorgenannten Beispiel wird zur Ermöglichung der Verfahrensdurchführung das nicht leitende Teil 1 partiell mit einer Metallbeschichtung 3 versehen, wogegen das metallische Teil zur Verhinderung einer vollständigen Bedeckung mit einer galvanisch abgeschiedenen Metallschicht auf einem Teil seiner Oberflächen mit einer Isolierschicht 7 versehen wird. Wiederum werden die Teile in eine feste Positionsbeziehung zueinander gebracht und unter vorbestimmten Prozessbedingungen in einem Elektrolysebad behandelt, wodurch die in Fig. 2B gezeigte Konfiguration gebildet wird. Hier sind, ebenso wie bei der ersten Ausführung, sämtliche metallischen Flächen mit einer abgeschiedenen Metallschicht bedeckt, die zugleich den ursprünglichen Spalt zwischen den Hüllenteilen 1, 1' ausfüllt und eine hermetisch dichte Verbindungsstelle liefert.

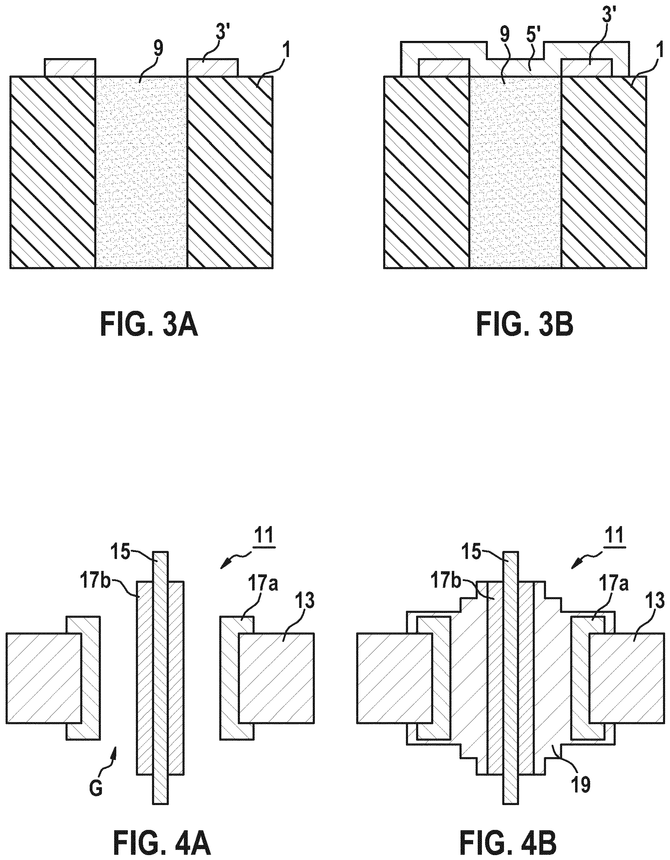

Fig. 3A und 3B zeigen eine Ausführung, bei der zwei nichtleitende Teile 1 an den einen der zugewandten Oberflächen vollflächig mittels eines Leitklebers 9 miteinander verbunden werden, nachdem ihre Oberseiten partiell mit einer lokalen Metallschicht (oder auch einem aufgepressten kleinen Metallteil) 3' versehen wurden. Die Teile werden hier nur teilweise in ein Elektrolysebad eingetaucht, weil nur ihre Oberseiten (einschließlich der freien Oberseite der Klebstoffschicht 9) mit einer abgeschiedenen Metallschicht versehen werden sollen, und im galvanischen Abscheidungsprozess bildet sich die Oberseiten-Metallisierung 5'. Diese bietet insbesondere eine Diffusionsbarriere auf der Klebstoffschicht 9 und macht die bereits vorher bestehende stoffschlüssige Verbindung hermetisch dicht. Bei dieser Konfiguration ist auch die Menge des für die Vorbehandlung und die galvanische Beschichtung verbrauchten Metalls deutlich geringer, und der galvanische Prozess kann schneller abgeschlossen werden. Weiterhin vereinfacht es die Ausrichtung der Teile zueinander während der galvanischen Abscheidung.

Während die obigen Beispiele ganz allgemeinen Charakter haben und in ihrer Anwendung nicht auf Hüllenteile einer elektronischen Baugruppe oder bestimmte Geräte beschränkt sind, zeigen Fig. 4A und 4B eine speziellere Konfiguration, nämlich diejenige einer Durchführung 11 eines implantierbaren elektromedizinischen Gerätes (nicht gezeigt). Hier sollen ein Titanflansch 1 und eine auf einem flüssigkristallinen Polymermaterial (LCP) basierende Leiterplatte 15, die den Flansch senkrecht zu seiner Erstreckungsebene durchstößt, hermetisch dicht miteinander verbunden werden.

Analog wie bei dem oben erläuterten ersten und zweiten Beispiel wird sowohl die Oberfläche des Titanflansches 13 als auch diejenige der LCP-Leiterplatte 15 partiell metallisiert, und zwar mittels eines aufgesetzten Goldringes 17a bzw. einer Goldbeschichtung 17b. Diese Vorbehandlung ist auch beim Titanflansch 13 erforderlich, weil dessen Oberfläche eine natürliche Korrosionsschicht trägt, die eine galvanische Abscheidung verhindern würde. In dem in Fig. 4B gezeigten Endzustand ist eine den Titanflansch und die LCP-Leiterplatte miteinander verbindende, den Ringspalt G zwischen diesen ausfüllende und die Komponenten hermetisch gegeneinander abdichtende Abscheide-Metallisierung 19 ausgebildet. Auch hier besteht eine erfindungsgemäße Abwandlung darin, die Komponenten vorher mittels eines Leitklebers stoffschlüssig miteinander zu verbinden, was insbesondere die Menge des zur Leitfähigmachung der einander zugewandten Oberflächen ansonsten benötigten Goldes reduziert und im Übrigen die Positionierung der Komponenten während der galvanischen Abscheidung vereinfacht.

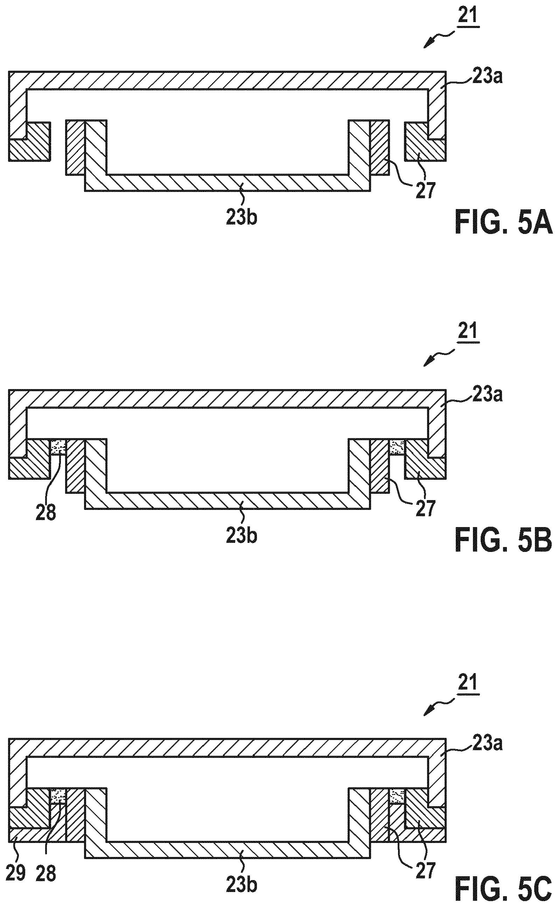

In Fig. 5A bis 5C ist eine weitere Ausführung der Erfindung schematisch in Art eines Längsschnittes dargestellt, bei der zwei Titan-Halbschalen 23a, 23b eines Implantatgehäuses 21 zusammengesetzt und abgedichtet werden. Vorab werden die Halbschalen 23a, 23b an den Rändern mit einer Gold-Beschichtung 27 versehen. Anschließend wird in den verbleibenden Spalt zwischen den Rändern der in ihrer Lagebeziehung fixierten Halbschalen 23a, 23b ein Strang Leitkleber 28 eingebracht und ausgehärtet. Im Elektrolysebad bildet sich anschließend auf den Oberflächen der partiellen Goldschicht und der freien Oberfläche des Leitklebers eine galvanisch abgeschiedene Metallschicht 29 aus, die das Implantat 23 hermetisch dicht macht. Die Abdichtung erfolgt erfindungsgemäß, ohne dass das Implantat höheren Temperaturen ausgesetzt wird, wie das beim üblicherweise angewandten Laserschweißverfahren nicht zu vermeiden ist.

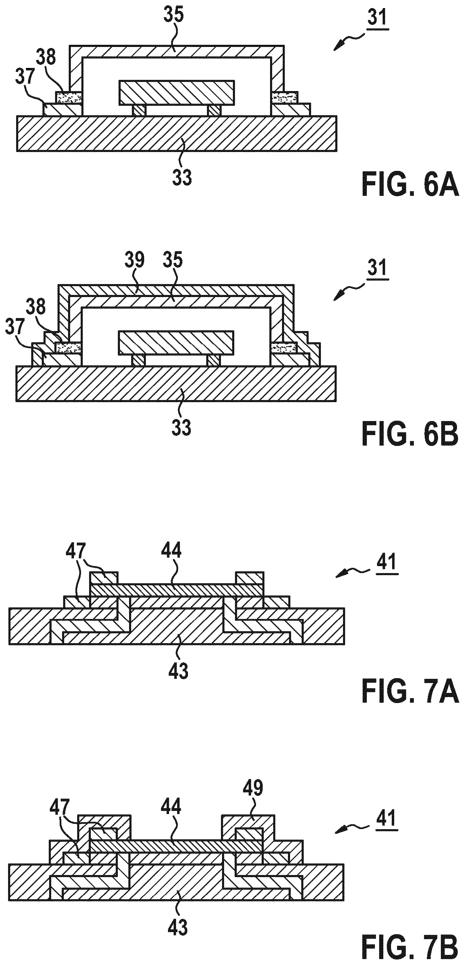

Fig. 6A und 6B zeigen, wiederum in schematischen Längsschnittdarstellungen, einen mit einem schützenden Deckel 35 versehenen Abschnitt eines Schaltungssubstrats 33 einer elektronischen Baugruppe 31. Während der Deckel 35 aus einem Metall mit metallisch blanker Oberfläche besteht, welches ohne Vorbehandlung galvanisch beschichtet werden kann, ist auf dem Schaltungssubstrat (etwa einer Leiterplatte auf Kunststoffbasis oder einem Keramiksubstrat) im Randbereich des aufzusetzenden Deckels wiederum eine Vorab-Metallisierung 37 erforderlich. Alternativ kann diese Metallisierung aus dem gleichen Material wie die Leiterbahnen auf dem Substrat ausgeformt werden und gleichzeitig mit dem normalen Leiterbild erzeugt werden. Auf diese Metallisierung 37 wird eine Klebstoffspur 38 aus einem leitfähigen Klebstoff aufgebracht, und auf diese wird der Deckel 35 aufgesetzt, so dass zunächst eine Verklebung des Deckels mit dem Schaltungssubstrat erfolgt. Anschließend erfolgt eine galvanische Metallisierung, die den abdeckenden und hermetisch dichten Metallüberzug 39 liefert.

Ist das Schaltungssubstrat selbst hermetisch dicht (was zum Beispiel bei Keramik der Fall ist) oder nahezu hermetisch dicht (beispielsweise bei einer LCP-basierten Leiterplatte), ist eine Diffusion aus der Umgebung in das Innere der abgedichteten Baugruppe nicht mehr bzw. kaum noch möglich. In Verbindung mit der den Deckel und die Klebespur überziehenden Metallisierung kann ein biokompatibles, hermetisches Package gefertigt werden, ohne dass thermische Prozesse bei über 100°C eingesetzt werden müssten.

Fig. 7A und 7B zeigen eine Abwandlung der zuletzt beschriebenen Ausführung bei einer Baugruppe 41, die wiederum auf einem Keramik- oder Kunststoff-Substrat 43 aufgebaut ist. Haupt-Funktionsbestandteil ist hier ein Sensorelement 44, mit entsprechenden (nicht gesondert bezeichneten) Anschlüssen, die durch das Schaltungssubstrat 43 hindurch verlaufen. Erforderlich ist hier eine hermetische Abdichtung des Umfangsrandes des Sensorelementes 44 auf dem Substrat 43. Hierzu werden zunächst sowohl auf den Umfangsrand des Sensorelementes als auch auf die benachbarten Bereiche des Substrats 43 eine galvanisch beschichtungsfähige Metallisierung 47 aufgebracht. Die nicht vorab metallisierten Oberflächenbereiche des Sensors 44 und des Schaltungssubstrats 43 sind wegen ihrer fehlenden Leitfähigkeit nicht galvanisch beschichtbar. Nach Behandlung im Elektrolysebad ergibt sich die in Fig. 7b dargestellte Konfiguration, mit einer das Sensorelement ringförmig partiell bedeckenden und umgebenden abgeschiedenen Metallschicht 49. Diese schafft eine hermetisch dichte Verbindung des Sensorelementes mit dem Schaltungssubstrat im Kantenbereich des Sensorelementes.

Die Ausführung der Erfindung ist nicht auf die oben beschriebenen Beispiele und Aspekte beschränkt, sondern ebenso in einer Vielzahl von Abwandlungen möglich, die im Rahmen fachmännischen Handelns liegen.

## Patentansprüche

1. Elektronische Baugruppe (31, 41), die eine aus mindestens zwei Hüllenteilen (1, 1'; 13, 15; 23a, 23b; 33, 35; 43, 44) zusammengefügte Umhüllung aufweist, wobei mindestens ein Spaltbereich (G) zwischen zwei einander benachbarten Hüllenteilen durch eine mindestens auf an den Spaltbereich angrenzende Abschnitte der benachbarten Hüllenteile galvanisch abgeschiedene, den Spaltbereich überbrückende Metallschicht (5; 19; 29; 39; 49) hermetisch dicht verschlossen ist,
wobei die einander benachbarten Hüllenteile (1; 23a; 23b; 33; 35) mit einem Klebstoff (9; 28; 38) verbunden sind, der den Spaltbereich (G) weitgehend ausfüllt, **dadurch gekennzeichnet, dass** der Klebstoff ein homogen leitender Klebstoff ist.

2. Elektronische Baugruppe nach Anspruch 1, ausgebildet als implantierbares medizinelektronisches Gerät, insbesondere Herzschrittmacher, Kardioverter, Cochlearimplantat oder Medikamentendosierpumpe.

3. Elektronische Baugruppe nach Anspruch 1, ausgebildet als hermetisch dichte und insbesondere EMI-dichte Komponente (31; 41) eines nicht insgesamt dichten elektronischen Gerätes.

4. Elektronische Baugruppe nach einem der vorangehenden Ansprüche, wobei mindestens eines der benachbarten Hüllenteile (1, 1'; 13, 15; 23a, 23b; 33, 35; 43, 44) ein Metallteil (1'; 23a, 23b; 35) mit unter der abgeschiedenen Metallschicht metallisch blanker Oberfläche ist.

5. Elektronische Baugruppe nach einem der vorangehenden Ansprüche, wobei mindestens eines der benachbarten Hüllenteile ein nicht leitender Teil (1; 15; 33; 43), etwa ein Kunststoffteil, insbesondere eine Leiterplatte, oder ein Keramikteil, insbesondere ein Dickschicht-Substrat, mit einer zusätzlichen Metallisierungsschicht (3; 3'; 37; 47) unter der abgeschiedenen Metallschicht (5; 39; 49) ist.

6. Elektronische Baugruppe nach einem der vorangehenden Ansprüche, wobei die galvanisch abgeschiedene Metallschicht (1, 1'; 13, 15; 23a, 23b; 33, 35; 43, 44) eine dichte, porenfreie Schicht bildet und insbesondere ein inertes Metall wie, z. B. Gold, Platin oder Palladium oder ein nicht inertes Metall wie z. B. Cu oder Ni aufweist.

7. Elektronische Baugruppe nach Anspruch 2 und einem der Ansprüche 4 bis 7, wobei die galvanisch abgeschiedene Metallschicht (19) auf einer Durchführung (11) des medizinelektronischen Gerätes vorgesehen ist und insbesondere den Spaltbereich (G) zwischen einem Flanschteil (13) und einem Leiterelement (15) der Durchführung überbrückt und abdichtet.

8. Elektronische Baugruppe nach Anspruch 7, wobei das Flanschteil (13) aus Titan besteht und partiell mit einem Edelmetall-Zusatzteil (17a) oder einer Edelmetallschicht versehen ist und das Leiterelement (15) ein Kunststoff- oder Keramiksubstrat aufweist und partiell mit einer Edelmetallschicht (17b) versehen ist.

9. Elektronische Baugruppe nach Anspruch 3 und einem der Ansprüche 4 bis 6, wobei eines der benachbarten Teile eine Leiterplatte (33) oder ein Dickschichtsubstrat und das andere Teil ein metallisches Deckelteil (35) ist und die Leiterplatte oder das Dickschichtsubstrat eine entsprechend der Öffnungskontur des Deckelteils konfigurierte partielle Metallbeschichtung (37) als Unterlage für die galvanisch abgeschiedene Metallschicht (39) aufweist.

10. Elektronische Baugruppe nach einem der vorangehenden Ansprüche, wobei die galvanisch abgeschiedene Metallschicht mehrere Metalle in Form einer Legierung und/oder einer Schichtfolge mit jeweils unterschiedlichen Metallen in jeder Teilschicht aufweist.

11. Elektronische Baugruppe nach einem der vorangehenden Ansprüche, wobei die galvanisch abgeschiedene Metallschicht eine Dicke im Bereich zwischen 0,1 und 100 µm aufweist.

12. Verfahren zur Herstellung einer elektronischen Baugruppe nach einem der vorangehenden Ansprüche, welches die Schritte aufweist:
Bereitstellen zweier für eine galvanische Abscheidung inhärent geeigneter oder vorbereiteter Hüllenteile(1, 1'; 13, 15; 23a, 23b; 33, 35; 43, 44),
Anordnen der Hüllenteile in einer vorbestimmten Lagebeziehung zueinander, die einen Spaltbereich (G) dazwischen einschließt, und Fixieren der Lagebeziehung der Hüllenteile, wobei das Fixieren der Lagebeziehung der Hüllenteile (1; 23a, 23b; 33, 35) deren Verkleben mittels eines homogen leitenden Leitklebers und mindestens teilweise Aushärten des Klebers (9; 28; 38) einschließt,
Verbringen der in ihrer Lagebeziehung fixierten Hüllenteile in ein Elektrolysebad und
Ausführen einer galvanischen Abscheidung im Elektrolysebad mit Verfahrensbedingungen, die die Abscheidung einer den Spaltbereich überbrückenden und hermetisch abdichtenden Metallschicht (5; 19; 29; 39; 49) gewährleisten.

13. Verfahren nach Anspruch 12, wobei das Bereitstellen für eine galvanische Beschichtung vorbereiteter Hüllenteile das mindestens partielle Aufbringen einer Metallisierung (3; 3'; 17b; 37; 47) oder Verbinden mit einem Edelmetallteil (17a) mindestens eines der Hüllenteile (1; 13; 15; 23a; 23b; 33; 43; 44) aufweist.

## Claims

1. An electronic assembly (31, 41), comprising an encasement joined from at least two casing parts (1, 1'; 13, 15; 23a, 23b; 33, 35; 43, 44), at least one gap region (G) between two casing parts neighboring each other being hermetically sealed by a metal layer (5; 19; 29; 39; 49) that is electrodeposited onto sections of the neighboring casing parts abutting the gap region and bridges the gap region,
the casing parts neighboring each other (1; 23a; 23b; 33, 35) being joined by way of an adhesive (9; 28; 38), which substantially fills the gap region (G), **characterized in that** the adhesive is a homogeneously conductive adhesive.

2. The electronic assembly according to claim 1, designed as an implantable electronic medical device, and in particular as a cardiac pacemaker, a cardioverter, a cochlear implant, or a drug dosing pump.

3. The electronic assembly according to claim 1, designed as a hermetically sealed and, in particular, an EMI-tight component (31; 41) of an electronic device that is not sealed in the overall.

4. The electronic assembly according to any one of the preceding claims, wherein at least one of the neighboring casing parts (1, 1'; 13, 15; 23a, 23b; 33, 35; 43, 44) is a metal part (1'; 23a, 23b; 35) comprising an uncoated surface beneath the deposited metal layer.

5. The electronic assembly according to any one of the preceding claims, wherein at least one of the neighboring casing parts is a non-conducting part (1; 15; 33; 43), such as a plastic part, and in particular a printed circuit board, or a ceramic part, and in particular a thick-film substrate, comprising an additional metallization layer (3; 3'; 37; 47) beneath the deposited metal layer (5; 39; 49).

6. The electronic assembly according to any one of the preceding claims, wherein the electrodeposited metal layer (1, 1'; 13, 15; 23a, 23b; 33, 35; 43, 44) forms a tight, pore-free layer and, in particular, comprises an inert metal, such as gold, platinum or palladium, or a non-inert metal, such as Cu or Ni.

7. The electronic assembly according to claim 2 and according to one of claims 4 to 7, wherein the electrodeposited metal layer (19) is provided on a feedthrough (11) of the electronic medical device and, in particular, bridges and seals the gap region (G) between a flange part (13) and a conductor element (15) of the feedthrough.

8. The electronic assembly according to claim 7, wherein the flange part (13) is made of titanium and partially provided with a noble metal additional part (17a) or a noble metal layer, and the conductor element (15) comprises a plastic or ceramic substrate and is partially provided with a noble metal layer (17b).

9. The electronic assembly according to claim 3 and one of claims 4 to 6, wherein one of the neighboring parts is a printed circuit board (33) or a thick-film substrate, and the other part is a metallic cover (35), and the printed circuit board or the thick-film substrate comprises a partial metal coating (37), which is configured in keeping with the opening contour of the cover and serves as a base for the electrodeposited metal layer (39).

10. The electronic assembly according to any one of the preceding claims, wherein the electrodeposited metal layer comprises multiple metals in the form of an alloy and/or a layer sequence comprising different metals in each sub-layer.

11. The electronic assembly according to any one of the preceding claims, wherein the electrodeposited metal layer has a thickness in the range between 0.1 and 100 µm.

12. A method for producing an electronic assembly according to any one of the preceding claims, comprising the following steps:
providing two casing parts (1, 1'; 13, 15; 23a, 23b; 33, 35; 43, 44) that are inherently suitable or prepared for electrodeposition;
arranging the casing parts in a predetermined relative position with respect to one another, which encloses a gap region (G) therebetween, and fixing the relative position of the casing parts, wherein the fixing of the relative position of the casing parts (1; 23a, 23b; 33, 35) comprises the bonding thereof by way of a homogeneously conductive adhesive and at least partial curing of the adhesive (9; 28; 38).
placing the casing parts fixed in the relative position thereof in an electrolysis bath; and carrying out an electrodeposition process in the electrolysis bath under method conditions that ensure the deposition of a metal layer (5; 19; 29; 39; 49) bridging and hermetically sealing the gap region.

13. The method according to claim 12, wherein the provision of the casing parts prepared for electrodeposition comprises the at least partial application of a metallization (3; 3'; 17b; 37; 47) or the joining to a noble metal part (17a) of at least one of the casing parts (1; 13; 15; 23a; 23b; 33; 43; 44).

## Revendications

1. Ensemble électronique (31, 41) qui présente une enveloppe assemblée à partir d'au moins deux parties d'enveloppe (1, 1' ; 13, 15; 23a, 23b ; 33, 35 ; 43, 45), où au moins une zone en fente (G) entre deux parties d'enveloppe voisines l'une de l'autre est fermée hermétiquement de manière étanche par au moins une couche métallique (5 ; 19 ; 29 ; 39 ; 49) déposée de manière galvanique sur les segments des parties d'enveloppe voisines à la limite de la zone de fente, surplombant la zone en fente, où les parties d'enveloppe (1 ; 23a ; 23b ; 33 ; 35) voisines les unes des autres sont reliées avec un adhésif (9 ; 28 ; 38) qui remplit largement la zone en fente (G), **caractérisé en ce que** l'adhésif est un adhésif conducteur homogène.

2. Ensemble électronique selon la revendication 1, conçu sous forme d'appareil électronique médical implantable, notamment de stimulateur cardiaque, de cardioverteur, d'implant cochléaire ou de pompe de dosage de médicament.

3. Ensemble électronique selon la revendication 1, conçu sous forme de composant (31 ; 41) étanche hermétiquement et notamment de composant d'EMI étanche d'un appareil électronique non totalement étanche.

4. Ensemble électronique selon l'une des revendications précédentes, dans lequel au moins une des parties d'enveloppe (1, 1' ; 13, 15 ; 23a, 23b ; 33, 35 ; 43, 44) voisines est une partie métallique (1' ; 23a, 23b ; 35) avec une surface exempte de métal sous la couche métallique déposée.

5. Ensemble électronique selon l'une des revendications précédentes, dans lequel au moins une des parties d'enveloppe voisines est une partie non conductrice (1 ; 15 ; 33 ; 43), notamment une partie en matière plastique, en particulier, un circuit imprimé ou une partie en céramique, en particulier un substrat en couche épaisse avec une couche de métallisation (3,3' ; 37 ; 47) supplémentaire en dessous de la couche métallique (5 ; 39 ; 49) déposée.

6. Ensemble électronique selon l'une des revendications précédentes, dans lequel la couche métallique (1, 1' ; 13, 15 ; 23a, 23b ; 33, 35 ; 43, 44) déposée de manière galvanique forme une couche étanche exempte de pores et présente en particulier un métal inerte, comme, par exemple, l'or, le platine ou le palladium ou un métal non inerte, comme, par exemple, le Cu ou le Ni.

7. Ensemble électronique selon la revendication 2 et l'une des revendications 4 à 7, dans lequel la couche métallique (19) déposée de manière galvanique est prévue sur un passage (11) de l'appareil médical électronique et assure un pontage et rend étanche en particulier la zone de fente (G) entre une partie d'épaulement (13) et un élément conducteur (15) du passage.

8. Ensemble électronique selon la revendication 7, dans lequel la partie en épaulement (13) est constituée de titane et est partiellement pourvue d'une pièce complémentaire en métal noble (17a) ou d'une couche de métal noble et l'élément conducteur (15) présente un substrat en matière plastique ou en céramique et est muni partiellement d'une couche de métal noble (17b).

9. Ensemble électronique selon la revendication 3 et l'une des revendications 4 à 6, dans lequel une des parties voisines est un circuit imprimé (33) ou un substrat en couche épaisse et l'autre partie est une partie de recouvrement (35) métallique et le circuit imprimé ou le substrat en couche épaisse présente un revêtement métallique (37) partiel conçu de manière correspondante au contour d'orifice de la partie de recouvrement sous forme de sous couche pour la couche métallique (39) déposée de manière galvanique.

10. Ensemble électronique selon l'une des revendications précédentes, dans lequel la couche métallique déposée de manière galvanique présente plusieurs métaux sous forme d'un alliage et/ou d'une série de couches avec chaque fois des métaux différents dans chaque couche partielle.

11. Ensemble électronique selon l'une des revendications précédentes, dans lequel la couche métallique déposée de manière galvanique présente une épaisseur dans une plage entre 0,1 et 100 µm.

12. Procédé de fabrication d'un ensemble électronique selon l'une des revendications précédentes, lequel présente les étapes :
de mise au point de deux parties d'enveloppe (1, 1' ; 13, 15 ; 23a, 23b ; 33, 35 ; 43, 44) appropriées de manière inhérente ou préparées pour un dépôt galvanique,
de disposition des parties d'enveloppe dans une organisation en couches prédéterminée les unes par rapport aux autres, qui renferme entre elles une zone en fente (G), et de fixation de l'organisation en couches des parties d'enveloppe, où la fixation de l'organisation en couches des parties d'enveloppe (1 ; 23a, 23b ; 33, 35) comporte leur collage au moyen d'un adhésif conducteur homogène et le durcissement au moins partiel de l'adhésif (9 ; 28 ; 38),
de mise en place des parties d'enveloppe fixées dans leur organisation en couches dans un bain d'électrolyse, et
d'exécution d'un dépôt galvanique dans le bain d'électrolyse avec des conditions de procédé qui assurent le dépôt d'une couche métallique (5 ; 19 ; 29 ; 39 ; 49) assurant le pontage de la zone en fente et étanche hermétiquement.

13. Procédé selon la revendication 12, dans lequel la mise au point de parties d'enveloppe préparées pour un revêtement galvanique présente l'au moins une application partielle d'une métallisation (3 ; 3' ; 17b ; 37 ; 47) ou la liaison d'au moins une des parties d'enveloppe (1 ; 13 ; 15; 23a ; 23b ; 33 ; 43 ; 44) avec une partie de métal noble (17a).
